# EUROPEAN PATENT SPECIFICATION

(11) **EP 4 114 344 B1**
(45) Date of publication and mention of the grant of the patent: **27.08.2025**
(21) Application number: 21704814.9
(22) Date of filing: 16.02.2021
(51) Int. Cl.: A61K 8/19, A61K 8/27, A61Q 11/00, A61K 8/25

(54) **TRANSPARENT DENTIFRICE COMPRISING ZINC**
TRANSPARENTE ZAHNPASTA MIT ZINK
DENTIFRICE TRANSPARENT COMPRENANT DU ZINC

(30) Priority: 03.03.2020 EP 20160749
(43) Date of publication of application: 11.01.2023
(73) Proprietor: Unilever IP Holdings B.V., 3013 AL Rotterdam (NL); Unilever Global IP Limited, Wirral, Merseyside CH62 4ZD (GB)
(72) Inventor: DABHOLKAR, Nandini, Sachin, Mumbai 400099 Andheri (IN); GOYAL, Richa, Sureshchand, 462003 Bhopal (IN); JOSHI, Aditi, Balkrishna, Mumbai 400099 Andheri (IN)
(74) Representative: Tansley, Sally Elizabeth
(86) International application number: PCT/EP2021/053744
(87) International publication number: WO 2021/175577

(56) References cited:
- WO-A1-2006/012988
- US-A1- 2007 183 989
- DATABASE GNPD [online] MINTEL; 2 April 2020 (2020-04-02), ANONYMOUS: "Intense Peppermint Fresh White Fluoride Dental Gel", XP055718281, retrieved from https://www.gnpd.com/sinatra/recordpage/7503943/ Database accession no. 7503943
- DATABASE GNPD [online] MINTEL; 22 October 2018 (2018-10-22), ANONYMOUS: "Complete Care Natural Toothpaste", XP055718286, retrieved from https://www.gnpd.com/sinatra/recordpage/6068517/ Database accession no. 6068517
- DATABASE GNPD [online] MINTEL; 22 October 2018 (2018-10-22), ANONYMOUS: "Complete Care Natural Toothpaste", XP055718286, retrieved from www.gnpd.com Database accession no. 6068517

## Description

### Technical Field of the Invention

The present invention relates to transparent gel dentifrice compositions.

### Background of the Invention

Transparent gel-type dentifrices are known in the art. Such dentifrices usually comprise silica as the abrasive as opposed to opaque toothpastes which primarily comprise chalk.

Dentifrices comprising compounds of zinc are also known in the art and have been disclosed in many patents. Formulation scientists have chosen to rely on highly water-soluble or at least somewhat water-soluble zinc salts. Zinc sulphate has been the ingredient of choice.

However, zinc sulphate is associated with an astringent mouthfeel.

Some sparingly water-soluble salts of zinc are reported as alternatives to zinc sulphate. Probably the most widely reported ingredient is zinc citrate.

The inclusion of such sparingly water-soluble zinc salts in transparent dentifrice gels presents some other technical problems. One of them is the ability of the gel to transmit light. In other words, such salts have the propensity to affect the transparent nature of the gels. However, some solutions are also known.

US 2007/0183989 A1 (Colgate) discloses gel toothpastes containing an active ingredient comprising a zinc citrate agent and a potassium salt, e.g. potassium citrate. It is disclosed that any other zinc salt, including water-soluble zinc salts could also be used. The exemplified compositions that contain zinc citrate do not contain adequate quantity of humectants.

US2019151209 A1 (Colgate) discloses a toothpaste comprising 0.01 to 2 wt% alginate, 0.1 to 2 wt% zinc compounds including zinc oxide and zinc citrate trihydrate, calcium carbonate abrasive at 38 to 50 wt %.

EP740932 A1 (Unilever, 1996) discloses a visually clear gel dentifrice comprising a zinc salt which is more water soluble than zinc citrate, an amino acid which can bind zinc and a low refractive index type abrasive silica.

WO2006012967 A1 (Unilever) discloses toothpastes comprising calcium carbonate as abrasive, water and a zinc salt, substantially all of which is in the form of water-solubilised zinc citrate. The toothpaste does not form zinc hydroxide upon storage in a toothpaste tube and hence no gassing due to the formation of carbon dioxide. Tripotassium citrate is necessary to solubilize the zinc citrate.

GB2052978 A (Unilever) discloses a toothpaste comprising zinc salts with glycine and a pH of from 4.5 to 8.0.

WO03090702 A1 (Unilever) discloses toothpastes having 0.001% to 20% of beads which include a zinc salt having a weight average particle size ranging from about 0.01 to about 5 mm. The bitter taste of zinc salts is improved by incorporating the zinc salt in the form of relatively large active beads.

US2018028423 A1 (Colgate) discloses oral care compositions comprising a basic amino acid in free or salt from (e.g., free form arginine); zinc oxide and zinc citrate; and a fluoride source. The inclusion of amino acid in the presence of zinc oxide and/or zinc citrate increases the antibacterial effect of oral care compositions at use.

US2017367948 A1 (Colgate) discloses oral care compositions comprising a first source of zinc which is zinc citrate, a second source of zinc which is zinc oxide or zinc lactate, a stannous ion source, and a polyphosphate he current formulations offer the advantage of robust microbial protection without significantly interfering with the stability of the oral care composition and by allowing for formulations which use comparable amounts of zinc and stannous which may have undesirable aesthetic qualities (e.g., poor taste). Zinc citrate is sparingly soluble in water and the concentration of solubilized zinc citrate in oral care compositions can vary over time. Solubilised form of zinc citrate is prepared by reacting zinc oxide with citric acid.

US2017258693 A1 (Colgate) discloses oral care composition comprising a source of zinc ions and a source of citrate ions, wherein the ratio of zinc ions to citrate ions is about 2:1 on a molar basis. The composition can include equimolar amounts of zinc chloride and zinc citrate which together can form an ionic complex ([Zn(citrate)]Cl).

US2013216485 A1 (Colgate) discloses toothpastes that comprise zinc citrate, silica, sorbitol and sodium hydroxide but the ratio between zinc citrate and sodium hydroxide is highly skewed in favour of sodium hydroxide.

WO0245678A2 (Colgate) discloses a stable aqueous oral care composition having limited astringency comprising a complex formed from zinc salt, anionic polycarboxylate polymer and a pyrophosphate salt.

US5188582 B1 (Chesebrough-Pond's, 1993) discloses a gel toothpaste that contains zinc citrate, sorbitol and 5% ethanol.

Get-type dentifrices generally contain abrasive silica as opposed to any calcium-based abrasive. Salts of zinc are often included in such compositions but the most preferred one is zinc sulphate which is highly water soluble. Use of highly water-soluble zinc salts may lead to incompatibility which could affect light transmittance of the gels. Some attempts to formulate such compositions with sparingly soluble zinc salts such as zinc citrate, have been disclosed as indicated hereinabove but they face at least some drawback, disadvantage or technical limitation.

### Summary of the Invention

We have determined that it is possible to formulate a visually clear and transparent gel dentifrice comprising abrasive silica and a sparingly-soluble compound of zinc such as zinc citrate, by striking a balance between the moles of zinc coming from the sparingly-soluble salt of zinc with the moles of hydroxide ions coming from sodium or potassium hydroxide. The pH should be between 6 and 8.5.

The solution is simpler than most solutions described in prior art where is necessary to use a combination of compounds zinc or to include an additional ingredient like glycine or to pre-solubilise the zinc citrate. Compositions in accordance with the invention are clear gels (high light transmittance) and are clinically efficacious without necessarily using any soluble form of zinc compounds e.g., zinc sulphate.

In accordance with the invention, disclosed is an aqueous alcohol-free dentifrice gel having pH 6 to 8.5 comprising:
a) 4 to 50 wt% abrasive silica;
b) 0.1 to 2.5 wt% of a sparingly water-soluble compound of zinc whose solubility at 20°C is 0.001 to 7 g/100 g water, wherein said sparingly water-soluble compound of zinc is at least one of zinc citrate, zinc oxide, zinc lactate, zinc gluconate, zinc glycinate, zinc oleate, zinc hydroxide and zinc peroxide,
c) 0.05 to 0.5 wt% sodium hydroxide or potassium hydroxide,
wherein the composition comprises 0.6 to 1.5 moles of hydroxide ions per mole of zinc ions in said sparingly water-soluble compound of zinc, wherein said composition comprises not more than 0,1% of water-soluble compound of zinc whose solubility at 20°C is greater than 1g/100 ml water, where said water-soluble compound of zinc sulphate, zinc chloride and zinc nitrate, wherein said dentifrice comprises a thickening system which comprises thickening silica and at least one of carboxymethyl cellulose, xanthan gum or guar gum.

All other aspects of the present invention will more readily become apparent upon considering the detailed description and examples which follow.

### Detailed Description

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

All references to the term/expression wt% or % by weight, shall mean percentage by weight of the composition, except where indicated otherwise.

The term aqueous implies that the compositions in accordance with the invention comprises water, preferably 10 to 60 wt%, more preferably 15 to 50 wt% and most preferably 20 to 40 wt% water. The water includes any that may come along with some other ingredients such as humectants, in particular, sorbitol.

Alcohol-free means that the compositions in accordance with the invention comprise not more than 1 wt%, preferably more than 0.5 wt% ethyl alcohol.

The term gel indicates physical appearance of the compositions of the invention. The gel compositions of the invention are transparent to light, i.e. they allow visible light to pass through. Clear gel toothpastes are popular with consumers. Undoubtedly, this is due, at least in part, to their appearance. On the other hand, there are some other types of dentifrice compositions that are not gels but are opaque. That is because such compositions usually contain opacifying ingredients such as chalk or other calcium-based abrasives. Therefore, it is preferred that the compositions of the invention do not comprise more than 5 wt%, preferably more than 2 wt% and more preferably more than 1 wt% calcium-based abrasive.

The method for assessing transparency involves use of a standard chart consisting of black symbols varying in size on a white background. This is the RIT Alphanumeric Resolution Test Object, RT 4-74, produced by Graphic Arts Research Center, Rochester Institute of Technology. Any other equivalent may also be used so long as the results do not differ drastically. The ability to discern the symbols clearly through a sample of product of standard thickness is measured. The symbols are assigned numbers from -12 to +13. The higher, more positive the number, the greater the clarity. The method can be applied to measurement of transparency of the dentifrice. If even the most prominent symbol cannot be readily defined through the layer of toothpaste, the gel dentifrice is considered cloudy and not transparent enough. In the practice of this invention, products with a numerical rating of about -12 or higher are considered transparent.

The dentifrice composition of the present invention is a clear gel toothpaste. By clear is meant having a translucency reading of about -12 or higher as the term translucency reading has been defined above, i.e., using RIT Alphanumeric Resolution Test Object RT4-74. The term "gel" as used herein includes not only those compositions which are gels in the strict technical sense but also gel-like compositions which have the same feeling and appearance of gels. Gel-like materials would include, for example, the pastes and creams which are familiar in the toothpaste art.

The dentifrice compositions of the invention preferably comprise a liquid or aqueous phase. The liquid phase of the composition is preferably formulated such, that its refractive index closely matches the refractive index of silica present as an abrasive. The liquid phase usually comprises water and a humectant, the latter usually being sorbitol or glycerol or a combination of the two. Water has RI of 1.333, sorbitol (70% aqueous solution) has an RI of 1.457 and glycerine an RI of 1.473.

Refractive index of the liquid phase can be calculated from the respective refractive indices of the constituting ingredients.

It is preferred that compositions of the invention comprise a total of 20 to 60 wt% humectant, more preferably 20 to 55 wt% and furthermore preferably 30 to 50 wt%. More preferably the humectant is at least one of glycerin, sorbitol, xylitol, butylene glycol or propylene glycol. Furthermore, preferably the dentifrice comprises sorbitiol and at least one other humectant selected from glycerin, xylitol, butylene glycol or propylene glycol, where sorbitol accounts for at least 50% of the total humectant content in said composition.

The compositions of the invention comprise 4 to 50 wt% abrasive silica. Preferably the abrasive silica is low refractive index silica with an apparent refractive index in the range of 1.41 to 1.47, preferably 1.435 to 1.445, preferably having a weight mean particle size of between 5 and 15 µm, a BET (nitrogen) surface area of between 10 and 100 m²/g and an oil absorption of about 70 to 150 cm³/100 g. Examples of suitable low refractive index abrasive silicas having an R.I. of between 1.435 and 1.445 are Tixosil 63 and 73 ex Rhone Poulenc; Sident 10 ex Degussa; Zeodent 113 ex Zeofinn; Sorbosil AC 77 ex Ineos having an R.I. of approximately 1.440. It is preferred that compositions of this invention comprise 4 to 20 wt% abrasive silica. It is further preferred that such silica is a combination of medium and high-abrasive silica. Their relative proportions may vary.

It is preferred that water activity of the compositions of the present invention is from 0.85 to 0.95 measured at about 25°C and refers to the equilibrium relative humidity or partial vapor pressure of the air above a product sample in a closed system divided by the relative humidity of air above pure water at the same temperature. Water activity can be expressed as % equilibrium relative humidity (% ERH) or Aw.% ERH=100×Aw.

Formulations that contain the same total concentration of water can have water activity values that differ dramatically depending on the hygroscopic characteristics of the other ingredients in the formula. Nearly all dentifrice or gel semi-solid formulations use polyhydric alcohols to help limit water loss over the shelf life of the product.

It is preferred that the sparingly water-soluble compound of zinc is at least one of zinc citrate, zinc oxide, zinc lactate, zinc gluconate, zinc glycinate, zinc oleate, zinc hydroxide and zinc peroxide. More preferably the salt is zinc citrate. A combination of two or more such compounds may also be used.

For example, solubility (g/100 g water) of some preferred sparingly water-soluble compounds of zinc is as follows:
- zinc citrate 6.11
- zinc lactate 1.4

In accordance with the invention, the dentifrice compositions comprise 0.05 to 0.5 wt% sodium or potassium hydroxide. Any one of the two or a combination, could be used, provided the amount is such that the composition comprises 0.6 to 1.5 moles of hydroxide ions per mole of zinc ions in the sparingly water-soluble compound.

In other words, the ratio of zinc ions (in the sparingly soluble salt) to hydroxide ions, is 1:0.6 to 1:1.5. When the ratio is outside the limits, the resultant compositions are hazy. Without wishing to be bound by theory it is believed that the clear and transparent nature of the dentifrice gel is attributable to an uncharacterised complex that forms between the zinc ions and the hydroxide ions. Such a phenomenon is not observed with highly water-soluble compound of zinc such as zinc sulphate, whose water-solubility is reported to be 57.7 g/100 g water at 25 °C as per
Lide, D.R. CRC Handbook of Chemistry and Physics 86TH Edition 2005- 2006. CRC Press, Taylor & Francis, Boca Raton, FL 2005, p. 4-96

Further, it is preferred that dentifrice compositions of the invention comprise less than 0.1 wt% of water-soluble compound of zinc whose solubility at 20°C is greater than 10 g/100 g water where said compound of zinc is zinc sulphate, zinc chloride and zinc nitrate.

Further preferably the dentifrice compositions of the invention comprise a thickening system which comprises thickening silica and at least one of carboxymethyl cellulose, xanthan gum or guar gum. Other binders and thickeners such as sodium carboxymethylcellulose, xanthan gum, gum arabic may also be included, as well as synthetic polymers such as polyacrylates and carboxyvinyl polymers such as Carbopol^{®}. It is preferred that dentifrice compositions of the invention comprise a thickening system which comprises thickening silica and at least one of carboxymethyl cellulose, xanthan gum or guar gum.

The dentifrice compositions of the invention may contain optional further ingredients such as cosmetically acceptable carriers like starch or sucrose. Small amounts of surfactants may also be included, such as anionic, nonionic and amphoteric surfactants.

Flavours such as peppermint and spearmint oils may also be included, as well as preservatives, colouring agents, pH adjusting agents, sweetening agents and so on.

Anti-bacterial agents may also be included such as Triclosan, chlorhexidine, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-4(4-chloro-6-bromophenol).

Polymeric compounds which can enhance the delivery of active ingredients such as anti-bacterial agents can also be included.

Anti-caries agents such as sodium- and stannous fluoride, aminefluorides, monosodiumfluorophosphate, casein, plaque buffers such as urea, pyruvates, arginine, small peptides, calcium lactate, calcium glycerophosphate, strontium polyacrylates may also be included. A preferred anti-caries agent is sodium monofluorophosphate. Other optional ingredients include vitamins such as Vitamin C, and plant extracts. Desensitising agents such as potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the dentifrice compositions may comprise anti-calculus agents such a alkali metal pyrophosphates, hypophosphite-containing polymers, organic phosphonates and phosphor-citrates.

Other optional ingredients that may be included are e.g. bleaching agents such as peroxy compounds e.g. potassium peroxy diphosphate, effervescing systems such as sodium bicarbonate/citric acid systems and colour change systems.

The dentifrices of the invention may also contain coloured particles suspended therein, e.g. coloured silica agglomerates or other coloured particles to impart a "speckled" appearance to the dentifrices. The dentifrices may also contain stripes of a coloured paste, to provide for a visually clear gel-type dentifrice with coloured stripes or a separate coloured phase

The dentifrice composition of the present invention is prepared by conventional methods of making oral care compositions. Such methods include mixing the ingredients under moderate shear and atmospheric pressure.

Typically, the composition will be packaged. In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out one to five times monthly.

The following examples are provided to facilitate an understanding of the present invention. The examples are not provided to limit the scope of the claims.

### Examples

### Example 1

Gel dentifrice compositions A to C were prepared by following the well-known method known in the art. Details are shown in Table 1. Compositions A and B were outside the scope of the invention, whereas C was as per the invention.

**Table 1**

| **Ingredients** | **Formulation Code/wt%** | | |
|---|---|---|---|
| | **A** | **B** | **C** |
| Sorbitol (70%) | 58.0 | 58 | 58.0 |
| Zinc citrate trihydrate | 0.7 | 1.0 | 2.0 |
| Thickening Silica | 8.3 | 8.3 | 8.3 |
| Medium Abrasive Silica | 9.0 | 9.0 | 9.0 |
| High Abrasive Silica | 1.6 | 1.6 | 1.6 |
| Sodium lauryl suphate | 2.3 | 2.3 | 2.3 |
| SCMC 9H | 0.7 | 0.7 | 0.7 |
| CAPB (30%) | 1.5 | 1.5 | 1.5 |
| NaOH solution (10%)** | 0.3 | 0.3 | 3.0 |
| Water and other minors to | 100.0 | 100.0 | 100.0 |
| Moles of Zn ions | 0.003 | 0.004 | 0.008 |
| Moles of OH ions | 0.00075 | 0.00075 | 0.0075 |
| Ratio of moles Zn⁺² to moles OH ions | 1:0.25 | 1:0.18 | 1:1.07 |

| | | | |
|---|---|---|---|
| **Note: the effective amount of NaOH in the composition is 1/10^{th} the amount stated in the table, due to dilution. | | | |

Applying the same method of preparing the compositions, a further series of compositions D to Q was prepared of which some were inside the scope of the invention and some were outside the scope.

Details of the amount of zinc citrate and sodium hydroxide present in these compositions are shown in Table 2. For wt% all other ingredients, reference is made to Table 1. The pH of all compositions was in the range of 6 to 6.5.

**Table 2**

| **Ref** | **Zinc citrate 3H₂O/grams** | **Moles of Zn⁺²** | **NaOH/grams** | **Moles/ OH ions** | **Ratio of moles Zn⁺² to moles OH ions** | **Observation** |
|---|---|---|---|---|---|---|
| E | 0.50 | 0.002 | 0.030 | 0.0007 | 3.183 | Turbid |
| F | 0.50 | 0.002 | 0.125 | 0.003 | 0.763 | Clear |
| H | 0.68 | 0.003 | 0.030 | 0.0007 | 4.328 | Turbid |
| I | 0.68 | 0.003 | 0.125 | 0.003 | 1.039 | Clear |
| K | 0.75 | 0.003 | 0.030 | 0.0007 | 4.774 | Turbid |
| L | 0.75 | 0.003 | 0.125 | 0.003 | 1.145 | Clear |
| N | 2.00 | 0.009 | 0.030 | 0.0007 | 12.732 | Turbid |
| O | 2.00 | 0.009 | 0.125 | 0.003 | 3.055 | Turbid |
| P | 2.00 | 0.009 | 0.300 | 0.0075 | 1.273 | Clear |

The data in table 2 clearly indicates the gel compositions were clear only when the ratio was between 0.6 and 1.5, i.e. only when the composition comprised 0.6 to 1.5 moles of hydroxide ions per mole of zinc ions in the sparingly water-soluble compound of zinc. All other compositions were turbid.

## Claims

1. An aqueous alcohol-free dentifrice gel having pH 6 to 8.5 and water activity from 0.85 to 0.95, comprising:
a) 4 to 50 wt% abrasive silica;
b) 0.1 to 2.5 wt% of a sparingly water-soluble compound of zinc whose solubility at 20°C is 0.001 to 7 g/100 ml water wherein said sparingly water-soluble compound of zinc is at least one of zinc citrate, zinc oxide, zinc lactate, zinc gluconate, zinc glycinate, zinc oleate, zinc hydroxide and zinc peroxide, and,
c) 0.05 to 0.5 wt% sodium hydroxide or potassium hydroxide,
wherein the composition comprises 0.6 to 1.5 moles of hydroxide ions per mole of zinc ions in said sparingly water-soluble compound of zinc, wherein said composition comprises not more than 0.1 wt% of water-soluble compound of zinc whose solubility at 20°C is greater than 1 g/100 ml water, where said water soluble compound of zinc is zinc sulphate, zinc chloride and zinc nitrate, wherein said dentifrice comprises a thickening system which comprise thickening silica and at least one of carboxymethyl cellulose, xanthan gum or guar gum.

2. A dentifrice as claimed in claim 1 wherein said composition comprises 20 to 60 wt% of a humectant.

3. A dentifrice as claimed in claim 1 or 2 wherein said composition comprises not more than 1 wt% of calcium-based abrasive.

4. A dentifrice as claimed in any of claims 1 to 3 wherein said humectant is at least one of glycerin, sorbitol, xylitol, butylene glycol or propylene glycol.

5. A dentifrice as claimed in any of claim 4 wherein said dentifrice comprises sorbitol and at least one other humectant selected from glycerin, xylitol, butylene glycol or propylene glycol, where sorbitol accounts for at least 50% of the total humectant content in said composition.

6. A dentifrice as claimed in any of claims 1 to 5 wherein refractive index of the abrasive silica is in the range of 1.41 to 1.47.

## Patentansprüche

1. Wässriges alkoholfreies Zahnpastagel mit einem pH-Wert von 6 bis 8,5 und einer Wasseraktivität von 0,85 bis 0,95, umfassend:
a) 4 bis 50 Gew.-% abrasives Siliciumdioxid;
b) 0,1 bis 2,5 Gew.-% einer schwer wasserlöslichen Zinkverbindung, deren Löslichkeit bei 20°C 0,001 bis 7 g/100 ml Wasser beträgt, wobei die schwer wasserlösliche Zinkverbindung mindestens eine der folgenden Verbindungen ist: Zinkcitrat, Zinkoxid, Zinklactat, Zinkgluconat, Zinkglycinat, Zinkoleat, Zinkhydroxid und Zinkperoxid, und
c) 0,05 bis 0,5 Gew.-% Natriumhydroxid oder Kaliumhydroxid,
wobei die Zusammensetzung 0,6 bis 1,5 Mol Hydroxidionen pro Mol Zinkionen in der schwer wasserlöslichen Zinkverbindung umfasst, wobei die Zusammensetzung nicht mehr als 0,1 Gew.-% wasserlösliche Zinkverbindung umfasst, deren Löslichkeit bei 20°C größer als 1 g/100 ml Wasser ist, wobei die wasserlösliche Zinkverbindung Zinksulfat, Zinkchlorid und Zinknitrat ist, wobei die Zahnpasta ein Verdickungssystem umfasst, das verdickendes Siliciumdioxid und mindestens eine der folgenden Verbindungen umfasst: Carboxymethylcellulose, Xanthangummi oder Guargummi.

2. Zahnpasta, wie im Anspruch 1 beansprucht, wobei die Zusammensetzung 20 bis 60 Gew.-% eines Feuchthaltemittels umfasst.

3. Zahnpasta, wie im Anspruch 1 oder 2 beansprucht, wobei die Zusammensetzung nicht mehr als 1 Gew.-% Schleifmittel auf Calciumbasis enthält.

4. Zahnpasta, wie in einem der Ansprüche 1 bis 3 beansprucht, wobei das Feuchthaltemittel mindestens eine der folgenden Verbindungen ist: Glycerin, Sorbit, Xylit, Butylenglycol oder Propylenglycol.

5. Zahnpasta, wie im Anspruch 4 beansprucht, wobei die Zahnpasta Sorbit und mindestens ein anderes Feuchthaltemittel umfasst, ausgewählt unter Glycerin, Xylit, Butylenglycol oder Propylenglycol, wobei Sorbit mindestens 50% des Gesamtgehalts an Feuchthaltemittel in der Zusammensetzung ausmacht.

6. Zahnpasta, wie in einem der Ansprüche 1 bis 5 beansprucht, wobei der Brechungsindex des abrasiven Siliciumdioxids in dem Bereich von 1,41 bis 1,47 liegt.

## Revendications

1. Gel dentifrice aqueux sans alcool ayant un pH de 6 à 8,5 et une activité de l'eau de 0,85 à 0,95, comprenant :
a) 4 à 50 % en poids de silice abrasive ;
b) 0,1 à 2,5 % en poids d'un composé du zinc modérément soluble dans l'eau dont la solubilité à 20 °C est de 0,001 à 7 g/100 ml d'eau, dans lequel ledit composé du zinc modérément soluble dans l'eau est au moins l'un parmi le citrate de zinc, l'oxyde de zinc, le lactate de zinc, le gluconate de zinc, le glycinate de zinc, l'oléate de zinc, l'hydroxyde de zinc et le peroxyde de zinc, et
c) 0,05 à 0,5 % en poids d'hydroxyde de sodium ou d'hydroxyde de potassium,
dans lequel la composition comprend 0,6 à 1,5 moles d'ions hydroxyde par mole d'ions zinc dans ledit composé du zinc modérément soluble dans l'eau, dans lequel ladite composition comprend au plus 0,1 % en poids de composé du zinc soluble dans l'eau dont la solubilité à 20°°C est supérieure à 1 g/100 ml d'eau, où ledit composé du zinc soluble dans l'eau est le sulfate de zinc, le chlorure de zinc et le nitrate de zinc, dans lequel ledit dentifrice comprend un système épaississant qui comprend une silice épaississante et au moins l'une parmi la carboxyméthylcellulose, la gomme xanthane et la gomme de guar.

2. Dentifrice selon la revendication 1, dans lequel ladite composition comprend 20 à 60 % en poids d'un humectant.

3. Dentifrice selon la revendication 1 ou 2, dans lequel ladite composition comprend au plus 1 % en poids d'abrasif à base de calcium.

4. Dentifrice selon l'une quelconque des revendications 1 à 3, dans lequel ledit humectant est au moins l'un parmi la glycérine, le sorbitol, le xylitol, le butylèneglycol et le propylèneglycol.

5. Dentifrice selon la revendication 4, dans lequel ledit dentifrice comprend du sorbitol et au moins un autre humectant choisi parmi la glycérine, le xylitol, le butylèneglycol et le propylèneglycol, où le sorbitol représente au moins 50 % de la teneur totale en humectants de ladite composition.

6. Dentifrice selon l'une quelconque des revendications 1 à 5, dans lequel l'indice de réfraction de la silice abrasive est situé dans la plage allant de 1,41 à 1,47.
